# EUROPÄISCHE PATENTSCHRIFT

(11) **EP 1 404 311 B1**
(45) Veröffentlichungstag und Bekanntmachung des Hinweises auf die Patenterteilung: **05.11.2008**
(21) Anmeldenummer: 02743052.9
(22) Anmeldetag: 17.05.2002
(51) Int. Cl.: A61K 31/205, A61K 31/155, A61P 17/02

(54) **WUNDBEHANDLUNGSMITTEL**
WOUND TREATMENT AGENT
PRODUIT DE TRAITEMENT DE PLAIES

(30) Priorität: 06.07.2001 DE 10132817
(43) Veröffentlichungstag der Anmeldung: 07.04.2004
(73) Patentinhaber: Prontomed GmbH, 32120 Hiddenhausen (DE)
(72) Erfinder: DAHLEN, Neithard, 35510 Butzbach (DE); KRAMER, Axel, Prof., 17493 Greifswald (DE)
(74) Vertreter: Tomerius, Isabel
(86) Internationale Anmeldenummer: PCT/EP2002/005486
(87) Internationale Veröffentlichungsnummer: WO 2003/004013

(56) Entgegenhaltungen:
- EP-A- 0 087 049
- EP-A- 0 513 637
- EP-A- 0 700 249
- WO-A-01/35743
- FR-A- 2 695 297
- US-A- 6 045 817

## Beschreibung

Die Erfindung betrifft eine Zusammensetzung zur Verwendung als Wundbehandlungsmittel, welche sich insbesondere als Wasch- oder Duschgel zur Dekontamination von Körperoberflächen, zum Lösen von Verkrustungen und Borken auf der Körperoberfläche und als Lösungsgel für Verbände eignet. Die erfindungsgemäße Zusammensetzung zur Verwendung als Wundbehandlungsmittel eignet sich besonders zum Einsatz im Krankenhausbereich.

Im Stand der Technik ist es bekannt, zum Waschen und Desinfizieren von Körperoberflächen ein Gel zu verwenden, welches aus Glycerin, Hydroxyethylcellulose und Chlorhexidin sowie Wasser besteht. Anstelle des Chlorhexidins kann auch eine quartäre Ammoniumverbindung zur Konservierung eingesetzt werden. Vor allem das Chlorhexidin hat jedoch den Nachteil, dass es eine zytotoxische Wirkung besitzt.

In der EP 0 700 249 B1 wird ein Antiinfektivum beschrieben, welches eine mikrobizide Wirkung gegen alle klinisch relevanten Keime und dabei eine gute Gewebeverträglichkeit besitzen soll. Der mikrobizide Wirkstoff ist Polyhexamethylenbiguanid (PHMB), welches mit einem Molekulargewicht von mindestens 2900 eingesetzt wird. Das in wässriger Lösung verwendete PHMB wird üblicherweise intravenös verabreicht. Außerdem wird die Verwendung einer verdünnten Lösung zum Desinfizieren offener Wunden beschrieben. Weiter wird erwähnt, dass der wässrigen Lösung des PHMB Tenside zugefügt werden können. Als einziges Beispiel eines Tensids wird Polyethylenglycol 4000 genannt, welches in erster Linie zur Verbesserung der Vernetzung dient.
Das in der EP 0 700 249 B1 beschriebene Antiinfektivum hat gegenüber anderen Antiinfektiva den Vorteil einer verringerten Gewebeschädigung. Seine Reinigungswirkung ist jedoch gering.

Es hat sich aber gezeigt, dass gerade bei chronischen Wunden die Wundheilung durch Wundbeläge stark verzögert wird. Diese Wundbeläge bestehen beispielsweise aus Wundexsudatresten, abgestoßenen und verdickten Fibrinbelägen, nekrotischem Gewebe und so genannten Zelltrümmern. Diese Beläge bieten gute Wachstumsbedingungen für Keime, die, wenn sie pathogen sind, zur Infektion der Wunde führen können.
Selbst bei Abwesenheit pathogener Keime ist es jedoch zur Beschleunigung der Wundheilung äußerst wichtig, Wundbeläge zu entfernen und die Wunde grundlegend zu reinigen und desinfizieren. Zum Beispiel können durch rechtzeitiges und gründliches Entfernen von Wundbelägen Wundverwachsungen und daraus resultierende Schmerzen vermieden werden. Es bestand daher ein Bedarf an einem Wundbehandlungsmittel, welches eine Wunde einerseits gründlich reinigt und von Wundbelägen befreit und andererseits Keime sicher abtötet, ohne allergisierend, sensibilisierend oder gewebeschädigend zu wirken.

Wie bereits erwähnt, ist PHMB als mikrobizider Wirkstoff mit guter Gewebeverträglichkeit für diesen Zweck grundsätzlich gut geeignet, wenn auch die Reinigungswirkung der bekannten Zusammensetzungen zu wünschen übrig lässt. Bekannt sind auch Wundreinigungszusammensetzungen in Form von Gelen, Tinkturen usw., denen jedoch das Problem der nicht zufriedenstellend gesicherten Konservierung und unzureichenden Wunddekontaminierung anhaftet.

Das der Erfindung zugrunde liegende technische Problem besteht also darin, ein Wundbehandlungsmittel mit guter Reinigungswirkung bei gleichzeitiger mikrobizider Wirkung anzugeben, welches sich zur Wundreinigung, Dekontamination von Körperoberflächen, zum Lösen von Verkrustungen und Verbänden und ähnlichen Anwendungen eignet, dabei aber möglichst frei von unerwünschten Nebenwirkungen ist.

Die Lösung gelingt mit der Zusammensetzung zur Verwendung als Wundbehandlungsmittel gemäß Anspruch 1. Bevorzugte Ausführungsformen sind den Unteransprüchen zu entnehmen. Die Erfindung betrifft außerdem die Verwendung der Zusammensetzung gemäß Anspruch 12.

Die Erfindung betrifft also eine Zusammensetzung zur Verwendung als Wundbehandlungsmittel, welche in wässriger Lösung Polyhexamethylenbiguanid in einer Konzentration von 0,01 bis 1,0 Gew.-% und wenigstens ein Tensid in einer Konzentratio von 0,01 bis 1,5 Gew.-% umfasst. Als Tensid wird ein Glycin-Derivat und/oder Sulfosuccinat und/oder Amid auf der Basis einer Fettsäure verwendet, wobei die Fettsäure 10 bis 18 Kohlenstoffatome aufweist.

Die hervorragende Wirksamkeit der erfindungsgemäßen Zusammensetzung basiert auf der speziellen Kombination von PHMB und gezielt ausgewählten Tensiden. Es hat sich nämlich im Verlauf der Untersuchungen, die zu dieser Erfindung führten, herausgestellt, dass die meisten Tenside zu einer deutlichen Reduzierung, wenn nicht gar zum völligen Verlust der mikrobiziden und konservierenden Eigenschaften des PHMB führen. Tendenziell ist es so, dass PHMB nur mit solchen Tensiden seine positiven Eigenschaften behält, die im Wundbehandlungsmittel in amphoterer oder nicht-ionogener Form vorliegen. Anionische Tenside beispielsweise bilden Komplexe mit PHMB, die zum Verlust der bioziden Eigenschaften führen.

Weiter hat sich gezeigt, dass - bei entsprechender Auswahl der Tenside - das PHMB als Konservierungsmittel im Wundbehandlungsmittel in deutlich geringerer Konzentration eingesetzt werden kann als andere Konservierungsmittel in vergleichbaren Zusammensetzungen. Hohe Konzentrationen von Konservierungsmittel führen jedoch zu einer deutlich stärkeren Belastung der behandelten Wunde und können letztlich zu starken Gewebeschädigungen führen. Dies gilt besonders für chronische Wunden, die wiederholt gereinigt werden müssen. Das erfindungsgemäße Wundbehandlungsmittel weist diese Nachteile praktisch nicht auf, da einerseits PHMB eine verglichen mit anderen keimabtötenden Substanzen ohnehin schon geringe Gewebetoxizität aufweist und es andererseits wegen der gezielten Auswahl des Tensids in der erfindungsgemäßen Zusammensetzung nur in einer sehr geringen Menge verwendet werden muss, um dennoch seine volle Wirkung zu entfalten.

Die Tenside, die erfindungsgemäß verwendet werden, sind Glycin-, Sulfosuccinat- und Amid-Derivate von Fettsäuren, die jeweils allein oder in beliebiger Kombination miteinander im Wundbehandlungsmittel vorliegen können. Die Sulfosuccinate sind ihrer Struktur nach an und für sich anionische Verbindungen. Wie schon erwähnt, werden aber erfindungsgemäß amphotere oder nicht-ionogene (neutrale) Tenside bevorzugt. Sulfosuccinate können jedoch bei entsprechendem pH-Wert auch in nichtionischer Form vorliegen, und in dieser Art und Weise werden sie erfindungsgemäß zweckmäßig verwendet.

Das Glycin-Derivat im erfindungsgemäßen Wundbehandlungsmittel ist bevorzugt ein Betain und insbesondere ein Amidoalkylbetain einer Fettsäure. Geeignete Fettsäuren für alle der erfindungsgemäß verwendeten Tenside besitzen 10 bis 18 Kohlenstoffatome und sind bevorzugt unverzweigt. Es können sowohl gesättigte als auch ungesättigte Fettsäuren verwendet werden. Beispiele besonders geeigneter Fettsäuren sind Undecylen-, Undecyl-, Laurin-, Stearin-, Ricinolsäure oder Kokosfettsäure.

In besonders bevorzugten Wundbehandlungsmitteln ist das Glycin-Derivat ein Undecylenamidoalkylbetain, Cocamidoalkylbetain, Lauramidoalkylbetain oder Ricinolamidoalkylbetain. Der Alkylrest ist bevorzugt Ethyl oder Propyl. Konkrete Beispiele sind Lauryldimethylaminoessigsäurebetain (auch als Laurylbetain bezeichnet), Lauramidopropylbetain (CAS-Nr. 4292-10-8), Cocamidopropylbetain (CAS-Nr. 61789-40-0) oder Ricinolamidopropylbetain (CAS-Nr. 86089-12-5). Das im Rahmen dieser Erfindung am meisten bevorzugte Tensid ist Undecylenamidopropylbetain (INCI-Bezeichnung; IUPAC: Dimethylcarboxymethyl-undecylensäureamidopropyl-ammonium-betain; CAS-Nr. 98510-75-9).

Zusätzlich zu dem Glycin-Derivat oder alternativ hierzu kann das erfindungsgemäße Wundbehandlungsmittel als Tensid ein Fettsäuresulfosuccinat oder ein Fettsäureamid enthalten. Letzteres wirkt üblicherweise auch als Verdickungsmittel und fördert die Rückfettung der Haut.
Der Fettsäurebestandteil dieser Tenside kann wie im Falle der Glycin-Derivate aufgebaut sein. Konkrete Beispiele dieser Tenside sind Undecylensäureamid, Undecylensäuresulfosuccinat oder Laurylsäuresulfosuccinat. Auch hier sind die Undecylensäure-Derivate besonders bevorzugt.

Wie bereits erwähnt, sind bereits relativ geringe Menge an PHMB zur Konservierung der erfindungsgemäßen Zusammensetzung zur Verwendung als Wundbehandlungsmittel ausreichend. Zweckmäßig wird das Polyhexamethylenbiguanid in einer Konzentration von 0,01 bis 1,0 Gew.-%, insbesondere 0,01 bis 0,3 Gew.-% und bevorzugt 0,1 Gew.-%, im Wundbehandlungsmittel verwendet.

Das Polyhexamethylenbiguanid kann als solches in der erfindungsgemäßen Zusammensetzung zur Verwendung als Wundbehandlungsmittel vorhanden sein, oder es kann in Form eines seiner Salze verwendet werden. Besonders geeignet ist hier das Hydrochlorid.
Zweckmäßig weist das Polyhexamethylenbiguanid, welches als Konservierungsmittel in der erfindungsgemäßen Zusammensetzung zur Verwendung als Wundbehandlungsmittel enthalten ist, ein mittleres Molekulargewicht von 2300 bis 3100 auf. Insbesondere liegt das mittlere Molekulargewicht bei 2300 bis unter 2900.

Das Tensid (bzw. das Tensidgemisch) ist bevorzugt in einer Konzentration von 0,01 bis 1,5 Gew.-%, insbesondere 0,03 bis 1 Gew.-%, bevorzugt 0,05 bis 0,4 Gew.-% und besonders bevorzugt 0,1 Gew.-%, vorhanden.

Die Zusammensetzung zur Verwendung als Wundbehandlungsmittel kann grundsätzlich in jeder für die Wundbehandlung bekannten und geeigneten Zubereitungsform verwendet werden. Als solche wären Salben, Tinkturen, Sprays, Waschlösungen, Gele usw. zu nennen. Bevorzugt wird die erfindungsgemäße Zusammensetzung zur Verwendung als Wundbehandlungsmittel in Form von Waschlösungen, Waschgelen oder gelartigen Wundauflagen eingesetzt. Eine Wundspüllösung besteht beispielsweise aus 99,8 Gew.-% Wasser, 0,1 Gew.-% Polyhexamethylenbiguanid und 0,1 Gew.-% Tensid, insbesondere Undecylensäureamidopropylbetain.

Wird die Zusammensetzung zur Verwendung als Wundbehandlungsmittel in Form eines Wundgels eingesetzt, enthält es die in medizinischen oder kosmetischen Gelen üblichen Zusätze. Für die erfindungsgemäßen Zusammensetzungen zur Verwendung als Wundbehandlungsmittel haben sich Zusätze von 1 - 15 Cew.-% Glycerin und 0,2 - 5 Cew.-% Hydroxyethylcellulose, insbesondere 5 - 12 Gew.-% Glycerin und 0,2 - 3 Gew.-% Hydroxyethylcellulose, zu den oben beschriebenen Gemischen als vorteilhaft erwiesen.

Eine Zusammensetzung zur Verwendung als Wundbehandlungsmittel in Form eines Gels besteht konkret beispielsweise aus 0,1 Gew.-% Polyhexamethylenbiguanid, 0,1 Gew.-% Tensid, insbesondere Undecylensäureamidopropylbetain, 8,6 Gew.-% Glycerin und 1,8 Gew.-% Hydroxyethylcellulose in Wasser, isotonischer Kochsalzlösung oder isotonischer Ringer-Lösung.

In allen erfindungsgemäßen Zusammensetzungen zur Verwendung als Wundbehandlungsmitteln können neben den genannten Komponenten die für Haut- und Wundreinigungsmittel sowie Antiinfektiva üblichen Hilfs- und Zusatzstoffe vorhanden sein. Beispielhaft können andere Konservierungsmittel, Farb-, Aroma- und Duftstoffe, Binde-, Feuchthalte- oder Benetzungsmittel, Konsistenzregler, Lösungsvermittler oder ähnliche genannt werden, welche in den üblichen Mengen zugefügt werden können.
Bevorzugt ist es jedoch, Anzahl und Konzentration der Hilfs- und Zusatzstoffe möglichst gering zu halten oder diese ganz zu vermeiden. Es versteht sich dabei von selbst, dass Stoffe, welche zu einer verminderten Wirkung der schon vorhandenen Komponenten führen oder auf das zu reinigende Gewebe nachteilig einwirken, möglichst zu vermeiden sind.

Als Lösungsmittel in der erfindungsgemäßen Zusammensetzung zur Verwendung als Wundbehandlungsmittel eignet sich entweder gereinigtes Wasser oder eine sonstige wässrige Lösung, wie sie üblicherweise im Bereich der Medizin eingesetzt wird. Geeignete Lösungsmittel sind beispielsweise Kochsalz- oder Ringer-Lösung. Zum Beispiel kann eine 0,4 bis 1,2 Gew.-%ige Kochsalzlösung verwendet werden. Besonders bevorzugt werden die Kochsalz- oder Ringer-Lösung in physiologischer Verdünnung eingesetzt. Im Falle von Lösungen umfasst die Erfindung sowohl die gebrauchsfertig verdünnten Lösungen als auch Konzentrate, die vor Verwendung verdünnt werden müssen.

Die bevorzugte Verwendung der Zusammensetzung zur Verwendung als Wundbehandlungsmittels ist die als Wasch- oder Duschlösung bzw. -gel, als Feuchthaltegel oder Feuchtwundauflage, als Lösung oder Lösungsgel zum Lösen von Verkrustungen oder Borken von Körperoberflächen oder Wunden oder zum Lösen von Verbänden und für den feuchten Verbandwechsel.

Besonders geeignet ist die erfindungsgemäße Zusammensetzung zur Verwendung als Wundbehandlungsmittel zur Behandlung chronischer Wunden, wie sie zum Beispiel bei Diabetikern oder bettlägerigen Patienten auftreten. Verdünnte wässrige Lösungen können zum Spülen oder Baden von Wunden oder zum Tränken und Befeuchten von Wundauflagen verwendet werden. Adhärierende Wundbandagen können durch Aufweichen mit der Lösung leicht von der Wunde abgelöst werden, ohne dass das Wundgewebe beschädigt wird. Beim Entfernen von Verbänden geschieht es häufig, dass bereits gebildete Verkrustungen beim Abnehmen des Verbandes unerwünscht wieder aufgerissen werden und sich so die Wundheilung verlangsamt. Auch hier schafft die erfindungsgemäße Zusammensetzung zur Verwendung als Wundbehandlungsmittel Abhilfe. Wird die Zusammensetzung zur Verwendung als Wundbehandlungsmittel auf den Verband aufgetragen, durchdringt sie diesen und löst ihn von den Verkrustungen der Wunde, so dass der Verband abgenommen werden kann, ohne dass die bereits gebildete Verkrustung beschädigt wird. Gleichzeitig wird verhindert, dass Bakterien oder dergleichen in die Wunde eingeschleppt werden.

Die erfindungsgemäße Zusammensetzung zur Verwendung als Wundbehandlungsmittel in Gelform kann als Feuchthaltegel oder als Feuchtwundauflage eingesetzt werden. Wundbeläge können so gelöst, die Wunde gereinigt und dekontaminiert werden. Ein Anwendungsbeispiel ist das Entfernen von Borken in Nasen von Patienten, die künstlich beatmet werden. Besonders geeignet ist das Gel auch zur Behandlung von Verbrennungen und Hautverpflanzungen, um die Wunde feucht und geschmeidig zu halten und keinen Nährboden für Bakterien oder sonstige Keime zu bieten. Die Auftragung kann unmittelbar auf die Wunde oder auf einen Wundverband erfolgen. Die Gelschicht verhindert zuverlässig die Re-Kontaminierung desinfizierter Wunden durch von außen eingetragene Keime.

Hauptanwendungsgebiet der erfindungsgemäßen Zusammensetzung zur Verwendung als Wundbehandlungsmittels ist, wie erwähnt, die Reinigung und Dekontaminierung von Wunden, die Wundkonditionierung und Wundsanierung. Hauptaugenmerk muss hierbei auf dem Entfernen von Wundbelägen liegen, die das Abheilen von Wunden erschweren und einen Nährboden für die Neuansiedlung pathogener Keime auf nicht infizierten Wunden bieten. Die gewünschte Reinigungswirkung wird erfindungsgemäß durch speziell ausgewählte Tenside erreicht, während das PHMB hauptsächlich der Konservierung der Tensidzusammensetzung und weniger der Dekontaminierung der Wunden dient. Die erfindungsgemäße Zubereitung schafft erstmals eine Zusammensetzung zur Verwendung als Wundbehandlungsmittel, die eine gute Reinigungswirkung mit guten bakteriostatischen und fungistatischen Eigenschaften verbindet, ohne dabei aber zu Gewebereizungen oder gar Gewebezerstörung zu führen. Wunden werden zuverlässig gereinigt und dekontaminiert, Geruchsbildung wird unterdrückt, und die Wundheilung kann gefördert werden.

Die Erfindung soll anhand eines Beispiels näher beschrieben werden.

### Beispiel 1

### Herstellung einer erfindungsgemäßen Wundspüllösung

Durch Vermischen der nachfolgend angegebenen Bestandteile auf übliche Weise wird eine Wundspüllösung zur Wundreinigung hergestellt.

| | |
|---|---|
| Steril filtriertes Trinkwasser | 99,8 g |
| Polyhexamethylenbiguanid ¹ | 0,1 g |
| Undecylenamidopropylbetain ² | 0,1 g |

| | |
|---|---|
| 1: 0,5 g Cosmocil® CQ (Avecia GmbH, Frankfurt/Main, DE) 2: 0,35 g REWOTERIC® AM B U 185 (Goldschmidt Rewo GmbH & Co. KG, Steinau, DE) | |

Das verwendete Polyhexamethylenbiguanid besaß ein mittleres Molekulargewicht von 2300 bis ca. 2900.

Die Wundspüllösung ist eine klare und geruchlose Flüssigkeit, die zum Spülen oder Baden akuter oder chronischer Wunden verwendet werden kann. Wundbeläge werden zuverlässig gelöst, ohne dass Nebenwirkungen wie Hautreizungen oder dergleichen zu beobachten sind.
Die Wundspüllösung eignet sich außerdem für den feuchten Verbandwechsel.

### Beispiel 2

### Herstellung eines erfindungsgemäßen Wundgels

Durch Vermischen der nachfolgend angegebenen Bestandteile auf übliche Weise wird ein Wundgel als Wundauflage hergestellt.

| | |
|---|---|
| Steril filtriertes Trinkwasser | 89,4 g |
| Polyhexamethylenbiguanid ¹ | 0,1 g |
| Undecylenamidopropylbetain ² | 0,1 g |
| Glycerin | 8,6 g |
| Hydroxyethylcellulose | 1,8 g |

| | |
|---|---|
| 1: 0,5 g Cosmocil® CQ (Avecia GmbH, Frankfurt/Main, DE) 2: 0,35 g REWOTERIC® AM B U 185 (Goldschmidt Rewo GmbH & Co. KG, Steinau, DE) | |

Es wird ein klares und geruchloses, leicht visköses Gel erhalten, das unmittelbar auf die kontaminierte Wunde oder auf einen Wundverband aufgetragen wird. Das Wundgel dringt auch in Wundtaschen problemlos ein und löst Wundbeläge wie Verkrustungen, Schorf oder tote Bakterien zuverlässig, ohne dass Nebenwirkungen wie Hautreizungen oder dergleichen zu beobachten sind. Wundverbände lassen sich leicht ablösen, ohne dass darunterliegendes Gewebe beschädigt wird.

## Patentansprüche

1. Zusammensetzung, welche in wässriger Lösung Polyhexamethylenbiguanid und wenigstens ein Tensid umfasst, worin
das Tensid ein Sulfosuccinat und/oder Amid auf der Basis einer Fettsäure und/oder ein Amidoalkylbetain einer Fettsäure ist, wobei die Fettsäure 10 bis 18 Kohlenstoffatome aufweist,
und das Polyhexamethylenbiguanid in einer Konzentration von 0,01 bis 1,0 Gew.-% und das Tensid in einer Konzentration von 0,01 bis 1,5 Gew.-% vorhanden ist,
zur Verwendung als Wundbehandlungsmittel.

2. Zusammensetzung gemäß Anspruch 1,
**dadurch gekennzeichnet,**
**dass** die Fettsäure unverzweigt ist.

3. Zusammensetzung gemäß Anspruch 2,
**dadurch gekennzeichnet,**
**dass** die Fettsäure Undecylen-, Undecyl-, Laurin-, Stearin-, Ricinolsäure oder Kokosfettsäure ist.

4. Zusammensetzung gemäß Anspruch 3,
**dadurch gekennzeichnet,**
**dass** das Amidoalkylbetain ein Undecylenamidoalkylbetain, Cocamidoalkylbetain, Lauramidoalkylbetain oder Ricinolamidoalkylbetain ist, wobei der Alkylrest bevorzugt Ethyl oder Propyl ist.

5. Zusammensetzung gemäß Anspruch 1,
**dadurch gekennzeichnet,**
**dass** das Tensid ein Fettsäuresulfosuccinat ist.

6. Zusammensetzung gemäß Anspruch 5,
**dadurch gekennzeichnet,**
**dass** das Tensid Undecylensäureamid, Undecylensäuresulfosuccinat oder Laurylsäuresulfosuccinat ist.

7. Zusammensetzung gemäß einem der Ansprüche 1 bis 6,
**dadurch gekennzeichnet,**
**dass** das Polyhexamethylenbiguanid in einer Konzentration von 0,01 bis 0,3 Gew.-% und bevorzugt 0,1 Gew.-% vorhanden ist.

8. Zusammensetzung gemäß einem der Ansprüche 1 bis 7,
**dadurch gekennzeichnet,**
**dass** das Tensid in einer Konzentration von 0,03 bis 1 Gew.-%, bevorzugt 0,05 bis 0,4 Gew.-% und besonders bevorzugt 0,1 Gew.-%, vorhanden ist.

9. Zusammensetzung gemäß Anspruch 7 oder 8, in Form einer Wundspüllösung,
**dadurch gekennzeichnet,**
**dass** sie aus 99,8 Gew.-% Wasser, 0,1 Cew.-% Polyhexamethylenbiguanid und 0,1 Gew.-% Tensid, insbesondere Undecylensäureamidopropylbetain, besteht.

10. Zusammensetzung gemäß Anspruch 7 oder 8, in Form eines Wundgels,
**dadurch gekennzeichnet,**
**dass** es 1 - 15 Gew.-% Glycerin und 0,2 - 5 Gew.-% Hydroxyethylcellulose, insbesondere 5 - 12 Gew.-% Glycerin und 0,2 - 3 Gew.-% Hydroxyethylcellulose, enthält.

11. Zusammensetzung gemäß Anspruch 10,
**dadurch gekennzeichnet,**
**dass** es besteht aus 0,1 Gew.-% Polyhexamethylenbiguanid, 0,1 Gew.-% Tensid, insbesondere Undecylensäureamidopropylbetain, 8,6 Gew.-% Glycerin und 1,8 Gew.-% Hydroxyethylcellulose in Wasser, isotonischer Kochsalzlösung oder isotonischer Ringer-Lösung.

12. Zusammensetzung gemäß einem der Ansprüche 1 bis 11 zur Verwendung als Wasch- oder Duschgel zur Wundreinigung, als Feuchthaltegel oder Feuchtwundauflage, als Lösungsgel zum Lösen von Verkrustungen oder Borken von Körperoberflächen oder Wunden oder zum Lösen von Verbänden, für den feuchten Verbandwechsel und zur Behandlung von Verbrennungen und Hautverpflanzungen.

## Claims

1. A composition which comprises polyhexamethylene biguanide and at least one surfactant, wherein the surfactant is a sulfosuccinate and/or an amide on the basis of a fatty acid and/or amidoalkyl betaine of a fatty acid, with the fatty acid having 10 to 18 carbon atoms, and the polyhexamethylene biguanide being present in a concentration of 0.01 to 0.1 percent by weight and the surfactant being present in a concentration of 0.01 to 1.5 percent by weight, for use as a wound-treatment agent.

2. A composition according to claim 1,
**characterized in that**
the fatty acid is unbranched.

3. A composition according to claim 2,
**characterized in that**
the fatty acid is undecylenic acid, undecylic acid, lauric acid, stearic acid, ricinoleic acid or coconut fatty acid.

4. A composition according to claim 3,
**characterized in that**
the amidoalkyl betaine is an undecylene amidoalkyl betaine, cocamidoalkyl betaine, lauramidoalkyl betaine or ricinoleamidoalkyl betaine, with the alkyl residue preferably being ethyl or propyl.

5. A composition according to claim 1,
**characterized in that**
the surfactant is a fatty acid sulfosuccinate.

6. A composition according to claim 5,
**characterized in that**
the surfactant is an undecylenic acid amide, undecylenic acid sulfosuccinate or lauryl acid sulfosuccinate.

7. A composition according to one of the claims 1 to 6,
**characterized in that**
the polyhexamethylene biguanide is present in a concentration of 0.01 to 0.3 percent by weight and preferably 0.1 percent by weight.

8. A composition according to one of the claims 1 to 7,
**characterized in that**
the surfactant is present in a concentration of 0.03 to 1 percent by weight, preferably 0.05 to 0.4 percent by weight and more preferably 0.1 percent by weight.

9. A composition according to claim 7 or 8, in the form of a wound-rinsing solution,
**characterized in that**
it consists of 99.8 percent by weight of water, 0.1 percent by weight of polyhexamethylene biguanide and 0.1 percent by weight of surfactant, especially undecylenic acid amidopropyl betaine.

10. A composition according to claim 7 or 8, in the form of a wound gel,
**characterized in that**
it contains 1 to 15 percent by weight of glycerine and 0.2 to 5 percent by weight of hydroxyethyl cellulose, especially 5 to 12 percent by weight of glycerine and 0.2 to 3 percent by weight of hydroxyethyl cellulose.

11. A composition according to claim 10,
**characterized in that**
it consists of 0.1 percent by weight of polyhexamethylene biguanide, 0.1 percent by weight of surfactant, especially undecylenic acid amidopropyl betaine, 8.6 percent by weight of glycerine, and 1.8 percent by weight of hydroxyethyl cellulose in water, isotonic saline solution or isotonic Ringer's solution.

12. A composition according to one of the claims 1 to 11, for use as a washing or shower gel for wound treatment, as a moisturizing gel or as a moist wound covering, as a dissolving gel for dissolving incrustations or scabs from body surfaces or wounds or for removing dressings and for changing moist dressings, and for treating burns and skin transplants.

## Revendications

1. Composition contenant en solution aqueuse du polyhexaméthylène biguanide et au moins un tensioactif, dans laquelle
le tensioactif est un sulfosuccinate et/ou un amide à base d'un acide gras et/ou une amidoalkylbétaïne d'un acide gras, lequel acide gras comporte entre 10 et 18 acides de carbone,
et le polyhexaméthylène biguanide est présent à une concentration de 0,01 à 1,0 % en poids et le tensioactif à une concentration de 0,01 à 1,5 % en poids,
destinée à être utilisée comme produit pour le traitement de plaies.

2. Composition selon la revendication 1,
**caractérisée en ce**
**que** l'acide gras n'est pas ramifié.

3. Composition selon la revendication 2,
**caractérisée en ce**
**que** l'acide gras est de l'acide undécylénique, undécylique, laurique, stéarique, ricinolique ou un acide gras de noix de coco.

4. Composition selon la revendication 3,
**caractérisée en ce**
**que** l'amidoalkylbétaïne est une amidoalkylbétaïne undécylénique, une amidoalkylbétaïne de noix de coco, une amidoalkylbétaïne laurique ou une amidoalkylbétaïne ricinolique, le radical alkyle étant de préférence un éthyle ou un propyle.

5. Composition selon la revendication 1,
**caractérisée en ce**
**que** le tensioactif est un sulfosuccinate d'acide gras.

6. Composition selon la revendication 5,
**caractérisée en ce**
**que** le tensioactif est un amide d'acide undécylénique, un sulfosuccinate d'acide undécylénique ou un sulfosuccinate d'acide laurylique.

7. Composition selon l'une des revendications 1 à 6,
**caractérisée en ce**
**que** le polyhexaméthylène biguanide est présent à une concentration de 0,01 à 0,3 % en poids et de préférence de 0,1 % en poids.

8. Composition selon l'une des revendications 1 à 7,
**caractérisée en ce**
**que** le tensioactif est présent à une concentration de 0,03 à 1 % en poids, de préférence de 0,05 à 0,4 % en poids et en particulier de 0,1 % en poids.

9. Composition selon la revendication 7 ou 8, sous la forme d'une solution pour l'irrigation de plaies,
**caractérisée en ce**
**qu'**elle se compose de 99,8 % en poids d'eau, 0,1 % en poids de polyhexaméthylène biguanide et 0,1 % en poids de tensioactif, en particulier d'amidopropylbétaïne d'acide undécylénique.

10. Composition selon la revendication 7 ou 8, sous la forme d'un gel pour plaies,
**caractérisée en ce**
**qu'**elle contient 1 à 15 % en poids de glycérine et 0,2 à 5 % en poids d'hydroxyéthylcellulose, en particulier 5 à 12 % en poids de glycérine et 0,2 à 3 % en poids d'hydroxyéthylcellulose.

11. Composition selon la revendication 10,
**caractérisée en ce**
**qu'**elle se compose de 0,1 % en poids de polyhexaméthylène biguanide, 0,1 % en poids de tensioactif, en particulier d'amidopropylbétaïne d'acide undécylénique, 8,6 % en poids de glycérine et 1,8 % en poids d'hydroxyéthylcellulose dans de l'eau, une solution isotonique de chlorure de sodium ou une solution de Ringer isotonique.

12. Composition selon l'une des revendications 1 à 11 destinée à être utilisée comme gel de lavage ou de douche pour le nettoyage de plaies, comme gel d'hydratation ou compresse humide de plaies, comme gel dissolvant pour la dissolution d'encroûtements ou de croûtes sur des surfaces du corps ou des plaies ou pour décoller des pansements, pour le renouvellement de pansements par voie humide et pour le traitement des brûlures et des greffes de peau.
